(19) Europäisches Patentamt
European Patent Office
Office européen des brevets

(11) Publication number: **0 339 406 A1**

(12) **EUROPEAN PATENT APPLICATION**

(21) Application number: 89106778.7

(51) Int. Cl.⁴: **C07D 215/56 , A61K 31/435**

(22) Date of filing: 15.04.89

(30) Priority: 19.04.88 JP 94679/88
20.06.88 JP 150340/88
14.11.88 JP 285640/88
23.01.89 JP 11987/89

(43) Date of publication of application:
02.11.89 Bulletin 89/44

(84) Designated Contracting States:
AT BE CH DE ES FR GB IT LI NL SE

(71) Applicant: **Hokuriku Pharmaceutical Co.,Ltd**
1-Chome, 3-14 Tatekawacho Katsuyamashi
Fukui(JP)

(72) Inventor: **Yasuo, Itoh**
3-11-14 Motomachi Katsuyamashi
Fukui(JP)
Inventor: **Hideo, Kato**
3-5-8 Kentoku Fukushi
Fukui(JP)
Inventor: **Eiichi, Koshinaka**
2-6-3 Asahicho Katsuyamashi
Fukui(JP)
Inventor: **Nobuo, Ogawa**
2-6-5 Asahicho Katsuyamashi
Fukui(JP)
Inventor: **Kazuya, Mitani**
2-2206 Shinbo Fukuishi
Fukui(JP)
Inventor: **Noriyuki, Yagi**
12-6-2 Inokeya Katsuyamashi
Fukui(JP)
Inventor: **Toshihiko, Yoshida**
19-35 Ootsuki Kamishiimura
Yoshidagun Fukui(JP)
Inventor: **Tomio, Suzuki**
19-15 Ootsuki Kamishiimura
Yoshidagun Fukui(JP)

(74) Representative: **Werner, Hans-Karsten, Dr. et al**
Deichmannhaus am Hauptbahnhof
D-5000 Köln 1(DE)

(54) **Quinoline-3-carboxylic acid derivatives, process for preparing the same, and composition exhibiting excellent antibacterial effect containing the same.**

(57) Quinoline-3-carboxylic acid derivatives represented by the formula (I);

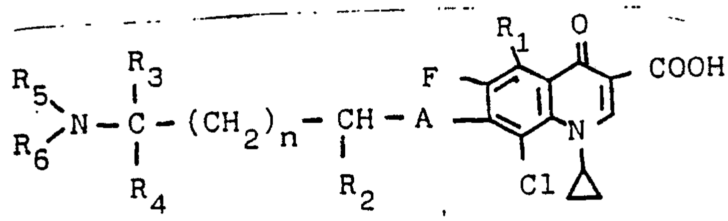

(I)

wherein $R_1$ is a hydrogen atom or an amino group; $R_2$ is a hydrogen atom or a lower-alkyl group, or $R_2$ combined with $R_4$ is an alkylene group having 1 through 4 methylenes; $R_3$ and $R_4$ are the same or different and each is a hydrogen atom or a lower-alkyl group, or $R_3$ combined with $R_4$ is an alkylene group having 2 through 6 methylenes; $R_5$ and $R_6$ are the same or different and each is a hydrogen atom or a lower-alkyl group, or $R_5$ combined with $R_2$ is an alkylene group having 1 through 4 methylenes, or $R_5$ combined with $R_3$, together with the neighboring nitrogen atom, is a 5- through 7- membered ring system which may be

EP 0 339 406 A1

substituted, or $R_5$ and $R_6$, together with the neighboring nitrogen atom, is a 5-through 7- membered ring system which may be substituted; A is an oxygen atom or a sulfur atom; n is selected from 0 through 3, and pharmacologically-acceptable salts thereof, which exhibit excellent antibacterial effect, a process for their preparation, pharmaceutical compositions thereof, and a method for the treatment of a subject afflicted with bacterial infections by administrating such a compound, are all disclosed.

# QUINOLINE-3-CARBOXYLIC ACID DERIVATIVES, PROCESS FOR PREPARING THE SAME, AND COMPOSITION EXHIBITING EXCELLENT ANTIBACTERIAL EFFECT CONTAINING THE SAME

## BACKGROUND OF THE INVENTION

### 1. Field for the Invention

The present invention relates to novel quinoline-3-carboxylic acid derivatives represented by the following general formula (I), pharmacologically-acceptable salts thereof, process for preparing the same, and pharmaceutical compositions exhibiting excellent antibacterial effect containing the same as active ingredient, and their use in the treatment of infections. The variables in the formula are described hereinafter.

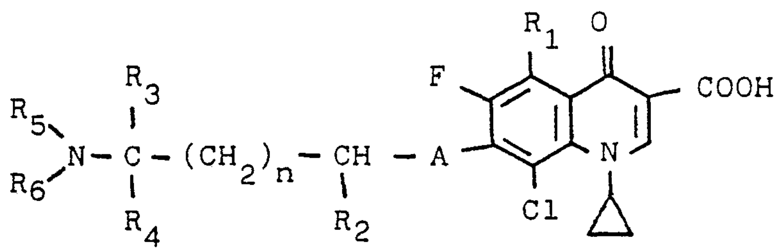

(I)

### 2. Description of the Prior Art

So far, as chemotherapeutic agents which have antibacterial effect, pyridonecarboxylic acids have been widely used. For example, nalidixic acid, piromidic acid, pipemidic acid and cinoxacine have been marketed for the clinical treatment of urinary tract infection, intestinal infection and cholangia infection. The most effective and widely used antibacterial agent among these is pipemidic acid (The Merck Index, 10th Edition, 7332) represented by the following formula:

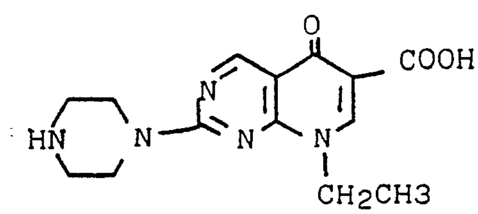

Recently, norfloxacin (The Merck Index, 10th Edition, 6541) was synthesized to improve the antibacterial effect of pipemidic acid and to expand its antibacterial spectrum. It has the following formula:

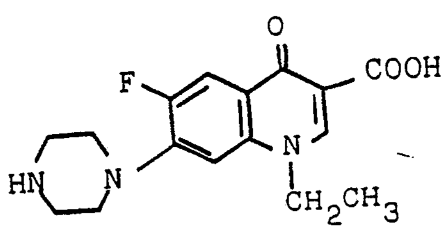

Further, relating to the antibacterial agents related to quinolines, ofloxacin (USAN and the USP dictionary of drug names, 399(1989)), which has the following formula;

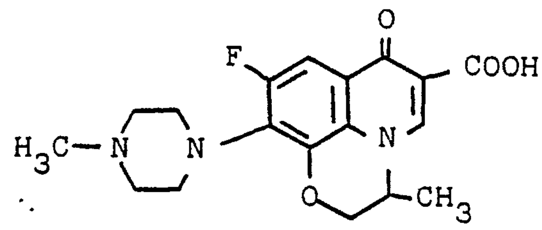

and ciprofloxacin hydrochloride (USAN and the USP dictionary of drug names, 131(1989)), which has the following formula;

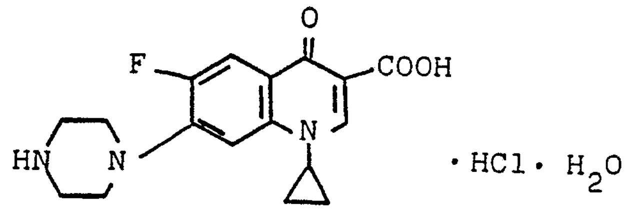

both having a broader antibacterial spectrum than norfloxacin, were synthesized in succession and used for the clinical treatment of infections such as pharynglaryngitis, pyelonephritis, cholecystitis, tympanitis, phlegmon, and blepharitis.

These antibacterial agents of pyridopyrimidines or quinolines are characterized by being 7-substituted by a 1-piperazinyl group, however, quinoline-3-carboxylic acid derivatives which are 7-substituted by an aminoalkoxy or aminoalkylthio group, according to the present invention, have been unknown.

The antibacterial agents which are quinolines, since the discovery of norfloxacin, have involved an epochal progress and it has been shown that they are adapted for various infections which are not limited to urinary tract infections. Their mechanism of action is the inhibition of DNA gyrase and these compounds do not produce a transmission of tolerance by the plasmid as do many antibiotics.

However, a steady increase of non-sensitive bacteria has been seen in clinics in recent years.

Because of these facts, it is difficult to believe that the antibacterial agents already developed are a complete answer, and a better antibacterial agent is always in demand for clinical use.

## 3.Summary of the Invention

It has now been found that novel quinoline-3-carboxylic acid derivatives, represented by the general formula (I):

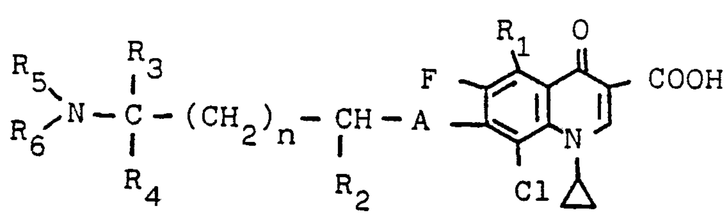

(I)

wherein $R_1$ is a hydrogen atom or an amino group; $R_2$ is a hydrogen atom or a lower-alkyl group, or $R_2$ combined with $R_4$ is an alkylene group having 1 through 4 methylene moieties; $R_3$ and $R_4$ may be the same or different and each is a hydrogen atom or a lower-alkyl group, or $R_3$ combined with $R_4$ is an alkylene group having 2 through 6 methylene moieties; $R_5$ and $R_6$ may be the same or different and each is a hydrogen atom or a lower-alkyl group, or $R_5$ combined with $R_2$ is an alkylene group having 1 through 4 methylene moieties, or $R_5$ combined with $R_3$, together with the neighboring nitrogen atom, is a 5- through 7- membered ring system which may be substituted, or $R_5$ and $R_6$, together with the neighboring nitrogen atom, is a 5- through 7- membered ring system which may be substituted; A is an oxygen atom or a sulfur atom; n is selected from 0 through 3, and pharmacologically-acceptable salts thereof, exhibit excellent antibacterial effect.

Further, according to the present invention, there are provided also a process for preparation of the

novel quinoline-3-carboxylic acid derivatives represented by the said formula (I), pharmaceutical compositions thereof, and a method of treating bacterial infections therewith.

## DETAILED DESCRIPTION OF THE INVENTION

In this invention, the lower-alkyl moiety represented by $R_2$, $R_3$, $R_4$, $R_5$ and $R_6$ in the said formula (I) is, for example, methyl, ethyl, n-propyl, isopropyl, n-butyl, isobutyl, sec-butyl, tert-butyl, and the like. The 5- or 7- membered ring which $R_3$ and $R_5$ together with the neighboring nitrogen atom constitute is, for example, 2-pyrrolidinyl, 1-methyl-2-pyrrolidinyl, 1-ethyl-2-pyrrolidinyl, 1-propyl-2-pyrrolidinyl, 2-piperidinyl, 1-methyl-2-piperidinyl, 1-ethyl-2-piperidinyl, and the like. The 5- or 7-membered ring which $R_5$ and $R_6$ together with the neighboring nitrogen atom constitute is, for example, 1-pyrrolidinyl, 1-piperidinyl, 4-methyl-1-piperidinyl, 3-methyl-1-piperidinyl, 2-methyl-1-piperidinyl, and the like. Typical examples of quinoline-3-carboxylic acid derivatives embraced by the present invention are:

7-(2-Aminoethoxy)-8-chloro-1-cyclopropyl-6-fluoro-1,4-dihydro- 4-oxoquinoline-3-carboxylic acid.
7-(2-Aminopropoxy)-8-chloro-1-cyclopropyl-6-fluoro-1,4-dihydro-4-oxoquinoline-3-carboxylic acid.
(R)-7-(2-Aminopropoxy)-8-chloro-1-cyclopropyl-6-fluoro-1,4-dihydro-4-oxoquinoline-3-carboxylic acid.
(S)-7-(2-Aminopropoxy)-8-chloro-1-cyclopropyl-6-fluoro-1,4-dihydro-4-oxoquinoline-3-carboxylic acid.
7-(2-Amino-2-methylpropoxy)-8-chloro-1-cyclopropyl-6-fluoro-1,4-dihydro-4-oxoquinoline-3-carboxylic acid.
7-[(1-Aminocyclopropyl)methoxy]-8-chloro-1-cyclopropyl-6-fluoro-1,4-dihydro-4-oxoquinoline-3-carboxylic acid.
7-(2-Amino-1-methylethoxy)-8-chloro-1-cyclopropyl-6-fluoro-1,4-dihydro-4-oxoquinoline-3-carboxylic acid.
trans-7-[(2-Amino-1-cyclopentyl)oxy]-8-chloro-1-cyclopropyl-6-fluoro-1,4-dihydro-4-oxoquinoline-3-carboxylic acid.
8-Chloro-1-cyclopropyl-6-fluoro-1,4-dihydro-7-[(1-methyl-3-pyrrolidinyl)oxy]-4-oxoquinoline-3-carboxylic acid.
8-Chloro-1-cyclopropyl-6-fluoro-1,4-dihydro-7-[(1-methyl-4-piperidinyl)oxy]-4-oxoquinoline-3-carboxylic acid.
7-[(2-Aminoethyl)thio]-8-chloro-1-cyclopropyl-6-fluoro-1,4-dihydro-4-oxoquinoline-3-carboxylic acid.
5-Amino-7-(2-aminopropoxy)-8-chloro-1-cyclopropyl-6-fluoro-1,4-dihydro-4-oxoquinoline-3-carboxylic acid.

Pharmacologically-acceptable salts of the compounds represented by the said formula (I) are acid addition salts or alkali addition salts. The former includes mineral acid salts such as the hydrochloride, hydrobromide, hydroiodide, nitrate, sulfate, phosphate, etc.; or organic acid salts such as the acetate, maleate, fumarate, citrate, oxalate, tartarate, methanesulfonate, p-toluenesulfonate, etc. The latter includes inorganic alkali salts such as the sodium, potassium, calcium, silver, zinc, lead, or ammonium salt, etc.; or organic base salts such as the ethanolamine or N,N-dialkylethanolamine salt, etc. The salts are all prepared in conventional manner, according to the skill of the art, and they may be converted to the free base and to each other, if desired, also in conventional manner.

Among the compounds represented by the said formula (I), compounds with an asymmetric carbon atom in the 7-substituent group are included. These compounds can take optically active forms, which are included in this invention. Morever, the compounds with two or more asymmetric carbon atoms can take geometrically different forms. Therefore, these geometrical isomers and their mixtures are included in this invention. The new quinoline-3-carboxylic acid derivatives represented by the said formula (I) can be prepared by various methods. In the first method, the compounds represented by the said formula (I) can be prepared by reacting 7-halogenoquinoline-3-carboxylic acid derivatives represented by the general formula (II),

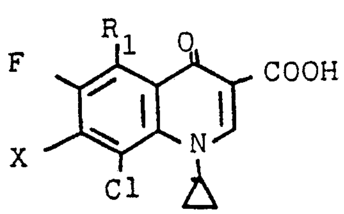

(II)

wherein $R_1$ has the same meaning as that described above, while X is a halogen atom, with alcohol or thioalcohol derivatives represented by the general formula (III);

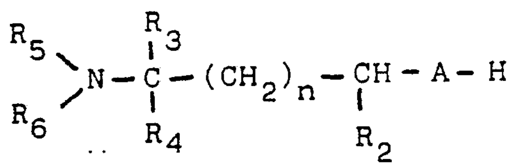

(III)

wherein $R_2$, $R_3$, $R_4$, $R_5$, $R_6$, A and n each has the same meaning as that described above, in the presence of a base in a solvent. The solvents used in this reaction can be any kind which does not inhibit the reaction. The solvents include, for example, aprotic polar solvents such as acetonitrile, N,N-dimethylformamide, N-methyl-2-pyrrolidone, dimethylsulfoxide, hexamethylphospholic triamide and the like, an aromatic solvent such as benzene, toluene and the like, a basic solvent such as pyridine, picoline, lutidine, collidine and the like, an alcoholic solvent such as methanol, ethanol, n-propanol, n-butanol, isoamyl alcohol and the like, or the mixture of these solvents.

The bases used in the present invention are, for example, sodium, sodium hydride, potassium hydride, sodium amide, sodium hydroxide, potassium hydroxide, sodium carbonate, potassium carbonate, and the like. The reaction is to be carried out at a temperature within the range from the ice-cooling temperature to the reflux temperature of the reaction solvent used.

The starting materials of this method, 7-halogenoquinoline-3-carboxylic acid derivatives represented by the said formula (II), are, for example, those already disclosed in Japanese Patent Publication (unexamined) No.1667/1986 and Japanese Patent Publication (unexamined) No.187459/1987. In the second method, the compounds represented by the said formula (I) can be prepared by reacting the compounds represented by the general formula (IV),

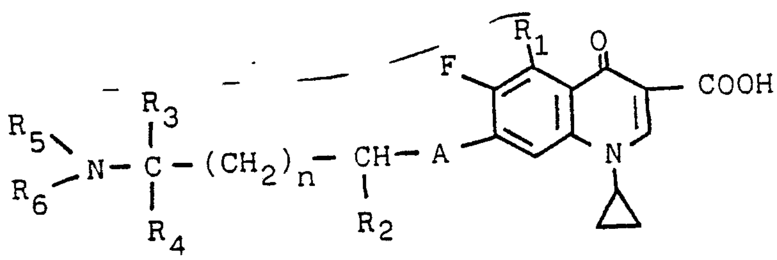

(IV)

wherein $R_1$, $R_2$, $R_3$, $R_4$, $R_5$, $R_6$, A and n each has the same meaning as that described above, with a chlorinating agent. The chlorinating agents used in the present invention are, for example, chlorine, sulfuryl chloride, and the like. The solvents used in this process are, for example, chloroform, dichloromethane, 1,2-dichloroethane, chlorosulfonic acid, acetic acid, and the like. The reaction is to be carried out at a temperature within the range from the ice-cooling temperature to the reflux temperature of the reaction solvent used.

In the third method, among the compounds represented by the said formula (I), the compounds wherein $R_5$ is a lower-alkyl group are prepared by reacting a compound represented by the said formula (I) wherein $R_5$ is a hydrogen atom with lower-halogenoalkyl compounds represented by the general formula (V),

$R_7 - Y$ (V)

wherein $R_7$ is a lower-alkyl group; Y is a halogen atom, in a solvent in the presence of a base, or with an aldehyde compound represented by the general formula (VI),

$R_8-\overset{O}{\overset{\|}{C}}-H$ (VI)

wherein $R_8$ is a hydrogen atom or a lower-alkyl group, in the presence of formic acid.

In this reaction, examples of the lower-halogenoalkyl compounds represented by the said formula (V) are methyl iodide, methyl bromide, ethyl iodide, ethyl bromide, propyl iodide, propyl bromide, butyl iodide, butyl bromide, and the like. The used solvents are, for example, N, N-dimethylformamide, acetone, ethanol, tetrahydrofuran, benzene, chloroform, and the like; and the bases are, for example, triethylamine, potassium carbonate, and the like. The reaction is to be carried out at a temperature within the range from the ice-cooling temperature to the reflux temperature of the reaction solvent used.

In this reaction, examples of the aldehyde compounds of the said formula (VI) are formaldehyde, acetaldehyde, propionaldehyde, and the like. Formaldehyde is used preferably in the form of its aqueous solution (formalin). In case of acetaldehyde or propionaldehyde, it is desirable to use nitrobenzene as a solvent for the reaction. The reaction is to be carried out at a temperature within the range from room temperature to 200°C A compound of the present invention represented by the said formula (I) can be administrated per os, e.g., in the form of pills or tablets, in which it may be present together with any of the usual pharmaceutical carriers, conventionally by compounding a compound of this invention together with a customary carrier or adjuvant, such as talc, magnesium stearate, starch, lactose, gelatin, any of numerous gums, or the like. Thus, in their most advantageous form, the compositions of this invention will contain a non-toxic pharmaceutical carrier in addition to the active ingredient of the present invention. Exemplary solid carriers are lactose, magnesium stearate, calcium stearate, starch, terra alba, dicalcium acacia, or the like.

Representative liquid carriers are peanut oil, sesame oil, olive oil, water, or the like. The active agents of this invention can be conveniently administered in such compositions containing active ingredient so as to eventually be within the dosage range illustrated hereinafter. Thus, a wide variety of pharmaceutical forms suitable for many modes of administration and dosages may be employed. For oral administration, the active ingredient and pharmaceutical carrier may, for example, take the form of a powder, granule, pill, tablet, capsule, lozenge, elixir, syrup, or other liquid suspension or emulsion. For parenteral administration, the composition may be in the form of a injectible, ophthalmic, or otic sterile solution. For intra-rectal administration, the composition may be in the form of a suppository.

The method of employing the compounds of this invention comprises internally or externally administering a compound of the invention, preferably orally or parenterally and preferably admixed with the pharmaceutical carrier, for example, in the form of any of the above compositions, or filled into a capsule, to alleviate conditions to be treated and symptoms thereof in a living animal body. Illustratively, it may be used in an amount of about 10 to about 1000 mg per day (divided into three parts), preferably in amount of 20 to 500 mg per day (divided into three parts) for oral dose, while parenteral dosages are usually less and ordinarily about one-half of the oral dose. The unit dose is preferably given a suitable number of times daily, typically three times.

The unit dose may vary depending upon the number of times given. Naturally, a suitable clinical dose must be adjusted in accordance with the condition, age, and weight of the patient, and it goes without saying that the enhanced activities of the compounds of the invention, together with their reduced side effects, also make them suitable for wide variations, and this invention therefore should not be limited by the exact ranges stated. The exact dosage, both unit dosage and daily dosage, will of course have to be determined according to established medical principles.

The novel compounds, quinoline-3-carboxylic acid derivatives represented by the said formula (I) and their pharmacologically-acceptable salts, which are obtained according to the methods described, have an antibacterial effect against both gram-positive and gram-negative microorganisms and are very useful as medicines.

The following experiment shows the excellent antibacterial effect of the present compounds (test compound number means Example compound number), while using norfloxacin which is a marketed product as a reference drug.

Experiment: Antibacterial Spectrum

Minimum inhibitory concentrations (MIC) were determined by the twofold agar dilution method (Chemotherapy 29(1), 76(1981)). Overnight cultures in Mueller-Hinton broth were suspended in buffered saline gelatin. One loopful of the bacterial suspension ($10^6$ or $10^8$ colony-forming units/ml) was incubated onto the test compound-containing plates. The plates were incubated for 18hrs. at 37°C. The MIC was the lowest concentration of the drug that inhibited visible growth. The results are shown in Table 1.

Table 1. Antibacterial Spectrum (Minimum concentration causing growth inhibition, $\mu g/m\ell$. $10^6$ cells/$m\ell$)

| Bacteria | Gram | Example 4 | Example 9 | Example 13 | Example 18 | Example 19 | Example 29 | Example 32 | Example 33 | Example 34 | Example 35 | Example 39 | Example 40 | Ref. Drug |
|---|---|---|---|---|---|---|---|---|---|---|---|---|---|---|
| Staphylococuss aureus FAD 209P JC-1 | + | 0.20 | 0.39 | 0.39 | 0.39 | 0.20 | 0.39 | 0.39 | 0.39 | 0.39 | 0.39 | 0.39 | 0.39 | 0.39 |
| Escherichia coli NIHJ JC-2 | − | 0.10 | 0.05 | 0.10 | 0.20 | 0.10 | 0.05 | 0.10 | 0.10 | 0.05 | 0.05 | 0.10 | 0.20 | 0.05 |
| Klebsiella pneumoniae PCI-602 | − | 0.05 | 0.05 | 0.025 | 0.05 | 0.025 | 0.025 | 0.025 | 0.05 | 0.025 | 0.025 | 0.05 | 0.05 | 0.05 |
| Serratia marcescens IAM 1184 | − | 0.20 | 0.20 | 0.20 | 0.20 | 0.20 | 0.10 | 0.39 | 0.20 | 0.10 | 0.10 | 0.20 | 0.39 | 0.10 |
| Pseudomonas aeruginosa IFO 3445 | − | 0.78 | 0.39 | 0.78 | 1.56 | 0.20 | 0.39 | 0.78 | 0.78 | 0.39 | 0.39 | 0.78 | 1.56 | 0.78 |
| Enterobacter cloaceae 963 | − | 0.10 | 0.10 | 0.10 | 0.20 | 0.10 | 0.10 | 0.10 | 0.20 | 0.10 | 0.05 | 0.10 | 0.20 | 0.20 |

The following References and Examples are given by way of illustration only and all not to be constructed as limiting.

Reference 1

1-Cyclopropyl-7-[2-(dimethylamino)ethoxy]-6-fluoro-1,4-dihydro-4-oxoquinoline-3-carboxylic acid

To a solution of 0.67g of 2-dimethylaminoethanol in 10 ml of N,N-dimethylformamide was added 0.30g of 60%-sodium hydride at room temperature with stirring. After stirring for 20 minutes, 0.50g of 1-cyclopropyl-6,7-difluoro-1,4-dihydro-4-oxoquinoline-3-carboxylic acid was added to the reaction mixture under ice-cooling, and then the reaction mixture was stirred at room temperature for 1 hour. The reaction mixture was adjusted with 10%-hydrochloric acid to pH3, and evaporated. Water was added to the residue, and the solution was adjusted with 10% aqueous sodium hydroxide solution to pH8. The whole was extracted with chloroform. The extract was dried and evaporated. The residue was converted into the hydrochloride in a usual manner, and the resulting salt was recrystallized from ethanol to give 0.32g of the desired compound as pale yellow needles, m.p.180-182 °C.

Analysis for $C_{17}H_{19}FN_2O_4$:

Calculated %: C,61.07; H,5.73; N,8.38.

Found %: C,61.02; H,5.69; N,8.47.

In the same manner as described in Reference 1, the compounds of References 2 to 14 were prepared.

Reference 2

7-(2-Aminopropoxy)-1-cyclopropyl-6-fluoro-1,4-dihydro-4-oxoquinoline-3-carboxylic acid

Pale yellow needles, m.p.210-214 °C (decomp.)($CHCl_3$-MeOH).

Analysis for $C_{16}H_{17}FN_2O_4 \cdot H_2O$:

Calculated %: C,56.80; H,5.66; N,8.28.

Found %: C,56.74; H,5.61; N,8.35.

Reference 3

7-(2-Aminobutoxy)-1-cyclopropyl-6-fluoro-1,4-dihydro-4-oxoquinoline-3-carboxylic acid

Yellow crystals, m.p.197-200 °C ($H_2O$).

Analysis for $C_{17}H_{19}FN_2O_4 \cdot H_2O$:

Calculated %: C,57.95; H,6.01; N,7.95.

Found %: C,57.91; H,5.63; N,7.97.

Reference 4

1-Cyclopropyl-7-[2-(dimethylamino)propoxy]-6-fluoro-1,4-dihydro-4-oxoquinoline-3-carboxylic acid

Pale yellow prisms, m.p.192-194 °C (MeOH).

Analysis for $C_{18}H_{21}FN_2O_4$:

Calculated %: C,62.06; H,6.08; N,8.04.

Found %: C,62.14; H,6.03; N,8.07.

Reference 5

7-(2-Amino-2-methylpropoxy)-1-cyclopropyl-6-fluoro-1,4-dihydro-4-oxoquinoline-3-carboxylic acid

Colorless crystals, m.p.197-200 °C ($CHCl_3$-MeOH).

Analysis for $C_{17}H_{19}FN_2O_4$:

Calculated %: C,61.07; H,5.73; N,8.38.

Found %: C,61.06; H,5.90; N,8.33.

Reference 6

7-(2-Amino-1-methylethoxy)-1-cyclopropyl-6-fluoro-1,4-dihydro-4-oxoquinoline-3-carboxylic acid hydrochloride

Colorless crystals, m.p.235-240 °C (EtOH-$CHCl_3$-$Et_2O$).

Calculated %: C,51.27; H,5.38; N,7.47.

Found %: C,51.36; H,5.24; N,7.50.

Reference 7

(R)-7-(2-Aminopropoxy)-1-cyclopropyl-6-fluoro-1,4-dihydro-4-oxoquinoline-3-carboxylic acid hydrochloride

The compound of Reference 7 was prepared using

(R)-2-amino-1-propanol [$[\alpha]_D^{22.3}$ +17.5° (c=1, MeOH)].
Colorless crystals, m.p.230-235 °C (decomp.)(MeOH-$Et_2O$).
IR spectrum $\nu$ (KBr) $cm^{-1}$: 1716 (COOH), 1634 (C=O)
NMR spectrum $\delta$ (DMSO-$d_6$) ppm: 0.82-1.60 (4H,m), 1.39 (3H,d,J=7Hz), 3.70-4.05 (1H,m), 4.51 (2H,d,J=5.5Hz), 7.86 (1H,d,J=7.5Hz), 8.03 (1H,d,J=11Hz), 8.69 (1H,s).

Specific rotation

$[\alpha]_D^{22.3}$ +7.8° (c=1,$H_2O$)

Reference 8

(S)-7-(2-Aminopropoxy)-1-cyclopropyl-6-fluoro-1,4-dihydro-4-oxoquinoline-3-carboxylic acid hydrochloride

The compound of Reference 8 was prepared using

(S)-2-amino-1-propanol [$[\alpha]_D^{22.3}$ -16.7° (c=1, MeOH)].
Colorless crystals, m.p.228-233° C (decomp.) (MeOH-$Et_2O$).
IR spectrum $\nu$ (KBr) $cm^{-1}$: 1716 (COOH), 1634 (C=O)
NMR spectrum $\delta$ (DMSO-$d_6$) ppm: 0.91-1.60 (4H,m), 1.40 (3H,d,J=7Hz), 3.70-4.05 (1H,m), 4.52 (2H,d,J=5.5Hz), 7.87 (1H,d,J=7.5Hz), 8.03 (1H,d,J=11Hz), 8.69 (1H,s).
Specific rotation

$[\alpha]_D^{22.3}$ -7.4° (c=1,$H_2O$)

Reference 9

1-Cyclopropyl-6-fluoro-1,4-dihydro-4-oxo-7-[2-(1-pyrrolidinyl)ethoxy]quinoline-3-carboxylic acid

Pale yellow plates, m.p.196-198° C (EtOH).

Analysis for $C_{19}H_{21}FN_2O_4$:

Calculated %: C,63.32; H,5.87; N,7.77.

Found %: C,63.34; H,6.13; N,7.78.

Reference 10

(S)-1-Cyclopropyl-6-fluoro-1,4-dihydro-4-oxo-7-[(2-pyrrolidinyl)methoxy]quinoline-3-carboxylic acid hydrochloride

The compound of Reference 10 was prepared using (S)-2-pyrrolidinemethanol [$[\alpha]_D^{20}$ +31.0° (c=1, toluene)].
Colorless needles, m.p.235-239° C (decomp.)(MeOH).

Analysis for $C_{18}H_{19}FN_2O_4 \cdot HCl \cdot H_2O$:

Calculated %: C,53.94; H,5.53; N,6.99.

Found %: C,54.13; H,5.31; N,7.04.

Specific rotation
$[\alpha]_D^{23}$ -2.0° (c=1,$H_2O$)

Reference 11

1-Cyclopropyl-6-fluoro-1,4-dihydro-4-oxo-7-[(3-pyrrolidinyl)oxy]quinoline-3-carboxylic acid

Pale yellow crystals, m.p.229-230.5 °C (decomp.)($H_2O$-MeOH).
IR spectrum $\nu$ (KBr) $cm^{-1}$: 1624 (C=O)
NMR spectrum $\delta$ ($CF_3COOD$) ppm: 1.27-2.00 (4H,m), 2.53-2.99 (2H,m), 3.71-4.45 (5H,m), 5.63-5.97 (1H,m), 8.15 (1H,d,J=6Hz), 8.39 (1H,d,J=10.5Hz), 9.42 (1H,s).

Reference 12

1-Cyclopropyl-6-fluoro-1,4-dihydro-7-[(1-methyl-3-pyrrolidinyl)oxy]-4-oxoquinoline-3-carboxylic acid

Pale gray prisms, m.p.184.5-185.5 °C ($CHCl_3$-EtOH).

Analysis for $C_{18}H_{19}FN_2O_4$:
Calculated %: C,62.42; H,5.53; N,8.09.
Found %: C,62.42; H,5.62; N,8.03.

Reference 13

1-Cyclopropyl-6-fluoro-1,4-dihydro-7-[(1-methyl-4-piperidinyl)oxy]-4-oxoquinoline-3-carboxylic acid

Colorless needles, m.p.243-246 °C ($H_2O$).

Analysis for $C_{19}H_{21}FN_2O_4 \cdot 1/2H_2O$:
Calculated %: C,61.78; H,6.00; N,7.58.
Found %: C,62.07; H,5.78; N,7.38.

Reference 14

1-Cyclopropyl-6-fluoro-1,4-dihydro-4-oxo-7-[(3-piperidinyl)oxy]quinoline-3-carboxylic acid hydrochloride

Pale yellow crystals, m.p.236-239 °C (MeOH).

Analysis for $C_{18}H_{19}FN_2O_4 \cdot HCl \cdot 1/2H_2O$:
Calculated %: C,55.18; H,5.40; N,7.15.
Found %: C,54.85; H,5.52; N,7.09.

Example 1

5-Amino-7-(2-aminoethoxy)-8-chloro-1-cyclopropyl-6-fluoro-1,4-dihydro-4-oxoquinoline-3-carboxylic acid hydrochloride

To a solution of 0.29g of 2-aminoethanol in 10 ml of N,N-dimethylformamide was added 0.19g of 60%-sodium hydride at room temperature with stirring for 10 minutes. The reaction mixture was added to a suspension of 0.50g of 5-amino-8-chloro-1-cyclopropyl-6,7-difluoro-1,4-dihydro-4-oxo-quinoline-3-carboxylic acid in 10ml of N,N-dimethylformamide under ice-cooling, and the reaction mixture was stirred at room temperature for 2 hours. The reaction mixture was adjusted with 10%-hydrochloric acid to pH2, and evaporated. Water was added to the residue, and the solution was adjusted with 10% aqueous sodium hydroxide solution to pH8. The whole was extracted with chloroform. The extract was dried and evaporated. The residue was converted into the hydrochloride in a usual manner and the resulting salt was recrystallized from a mixture of methanol and ether to give 0.03g of the desired compound as yellow crystals, m.p.239-244° C (decomp.).

Analysis for $C_{15}H_{15}ClFN_3O_4 \cdot HCl$:

Calculated %: C,45.94; H,4.11; N,10.71.
Found %: C,45.60; H,4.49; N,10.73.

In the same manner as described in Example 1, the compounds of Examples 2 to 18 were prepared.

Example 2

5-Amino-8-chloro-1-cyclopropyl-6-fluoro-1,4-dihydro-7-[2-(methylamino)ethoxy]-4-oxoquinoline-3-carboxylic acid hydrochloride

yellow crystals, m.p.225-230° C (decomp.)(MeOH-$Et_2O$).

Analysis for $C_{16}H_{17}ClFN_3O_4 \cdot HCl$:

Calculated %: C,47.31; H,4.47; N,10.34.
Found %: C,47.20; H,4.54; N,10.37.

Example 3

5-Amino-8-chloro-1-cyclopropyl-7-[2-(dimethylamino)ethoxy]-6-fluoro-4-oxoquinoline-3-carboxylic acid hydrochloride

yellow needles, m.p.224-229° C (decomp.)(MeOH-$Et_2O$).

Analysis for $C_{17}H_{19}ClFN_3O_4 \cdot HCl$:

Calculated %: C,48.59; H,4.80; N,10.00.
Found %: C,48.40; H,5.09; N, 9.87.

Example 4

5-Amino-7-(2-aminopropoxy)-8-chloro-1-cyclopropyl-6-fluoro-1,4-dihydro-4-oxoquinoline-3-carboxylic acid hydrochloride

yellow crystals, m.p.225-230 °C (decomp.)(MeOH-$Et_2O$).

Analysis for $C_{16}H_{17}ClFN_3O_4 \cdot HCl \cdot 1/2H_2O$:

Calculated %: C,46.28; H,4.61; N,10.12.

Found %: C,46.07; H,4.71; N, 9.94.

Example 5

5-Amino-7-(2-amino-2-methylpropoxy)-8-chloro-1-cyclopropyl-6-fluoro-1,4-dihydro-4-oxoquinoline-3-carboxylic acid hydrochloride

yellow crystals, m.p.222-227 °C (decomp.)(MeOH-$Et_2O$).

Analysis for $C_{17}H_{19}ClFN_3O_4 \cdot HCl \cdot H_2O$:

Calculated %: C,46.59; H,5.06; N, 9.59.

Found %: C,46.59; H,5.12; N, 9.23.

Example 6

(S)-5-Amino-8-chloro-1-cyclopropyl-6-fluoro-1,4-dihydro-7-[(1-methyl-2-pyrrolidinyl)methoxy]-4-oxoquinoline-3-carboxylic acid hydrochloride

The compound of Example 6 was prepared using (S)-1-methyl-2-pyrrolidinemethanol [$[\alpha]_D^{23}$ -50.2° (c = 1, MeOH)].
Yellow crystals, m.p.223-228 °C (decomp.)(MeOH-$Et_2O$).

Analysis for $C_{19}H_{21}ClFN_3O_4 \cdot HCl \cdot 1/2H_2O$:

Calculated %: C,50.12; H,5.09; N,9.23.

Found %: C,50.25; H,5.15; N,9.25.

Specific rotation
$[\alpha]_D^{24}$ -3.0° (c = 1,$H_2O$)

Example 7

5-Amino-8-chloro-1-cyclopropyl-6-fluoro-1,4-dihydro-7-[(1-methyl-3-pyrrolidinyl)oxy]-4-oxoquinoline-3-carboxylic acid hydrochloride

yellow crystals, m.p.263-268°C (decomp.)(MeOH-$Et_2O$).

Analysis for $C_{18}H_{19}ClFN_3O_4 \cdot HCl \cdot 1/2H_2O$:

Calculated %: C,48.99; H,4.80; N, 9.52.

Found %: C,49.26; H,4.93; N, 9.31.

Example 8

5-Amino-7-(2-amino-1-methylethoxy)-8-chloro-1-cyclopropyl-6-fluoro-1,4-dihydro-4-oxoquinoline-3-carboxylic acid hydrochloride

yellow crystals, m.p.260-265°C (decomp.)(MeOH-$Et_2O$).

Analysis for $C_{16}H_{17}ClFN_3O_4 \cdot HCl$:

Calculated %: C,47.31; H,4.47; N, 10.34.

Found %: C,47.15; H,4.69; N, 10.15.

Example 9

7-(2-Aminoethoxy)-8-chloro-1-cyclopropyl-6-fluoro-1,4-dihydro-4-oxoquinoline-3-carboxylic acid hydrochloride

Pale brown crystals, m.p.205-208°C (MeOH).

Analysis for $C_{15}H_{14}ClFN_2O_4 \cdot HCl \cdot 1/2H_2O$:

Calculated %: C,46.65; H,4.18; N, 7.25.

Found %: C,46.46; H,4.20; N, 7.23.

Example 10

8-Chloro-1-cyclopropyl-6-fluoro-1,4-dihydro-7-[2-(methylamino)ethoxy]-4-oxoquinoline-3-carboxylic acid hydrochloride

Colorless prisms, m.p.208-210°C (decomp.)(MeOH).

Analysis for $C_{16}H_{16}ClFN_2O_4 \cdot HCl$:

Calculated %: C,49.12; H,4.38; N, 7.16.

Found %: C,49.23; H,4.34; N, 7.14.

Example 11

7-[(2-Aminopentyl)oxy]-8-chloro-1-cyclopropyl-6-fluoro-1,4-dihydro-4-oxoquinoline-3-carboxylic acid hydrochloride

Colorless crystals, m.p.207-208 °C ($EtOH-Et_2O$).

Analysis for $C_{18}H_{20}ClFN_2O_4 \cdot HCl$:

Calculated %: C,51.56; H,5.05; N, 6.68.

Found %: C,51.35; H,5.08; N, 6.67.

Example 12

7-(2-Amino-3-methylbutoxy)-8-chloro-1-cyclopropyl-6-fluoro-1,4-dihydro-4-oxoquinoline-3-carboxylic acid hydrochloride

Pale pink crystals, m.p.210-213 °C ($MeOH-Et_2O$).

Analysis for $C_{18}H_{20}ClFN_2O_4 \cdot HCl$:

Calculated %: C,51.56; H,5.05; N, 6.68.

Found %: C,51.25; H,5.17; N, 6.62.

Example 13

7-[(1-Aminocyclopropyl)methoxy]-8-chloro-1-cyclopropyl-6-fluoro-1,4-dihydro-4-oxoquinoline-3-carboxylic acid hydrochloride

Pale orange crystals, m.p.206-208 °C (decomp.)($EtOH-Et_2O$)
IR spectrum $\nu$ (KBr) $cm^{-1}$: 1732 (COOH), 1614 (C=O)
NMR spectrum $\delta$ (DMSO-$d_6$) ppm: 0.88-1.40 (8H,m), 4.26-4.59 (1H,m), 4.39 (2H,s), 8.12 (1H,d,J=10.5Hz), 8.80 (2H,br-s), 8.84 (1H,s)

Example 14

7-[(1-Aminocyclopentyl)methoxy]-8-chloro-1-cyclopropyl-6-fluoro-1,4-dihydro-4-oxoquinoline-3-carboxylic acid hydrochloride

Pale yellow crystals, m.p.222-225 °C (decomp.)(MeOH-AcOEt).

Analysis for $C_{19}H_{20}ClFN_2O_4 \cdot HCl$:

Calculated %: C,52.91; H,4.91; N, 6.50.

Found %: C,52.51; H,5.20; N, 6.21.

Example 15

(S)-8-Chloro-1-cyclopropyl-6-fluoro-1,4-dihydro-7-[(1-methyl-2-pyrrolidinyl)methoxy]-4-oxoquinoline-3-carboxylic acid hydrochloride

The compound of Example 15 was prepared using (S)-1-methyl-2-pyrrolidinemethanol [$[\alpha]_D^{23}$ -50.2° (c = 1, MeOH)]
Pale yellow needles, m.p.177.5-178.5° C (EtOH).

Analysis for $C_{19}H_{20}ClFN_2O_4 \cdot HCl$:

| | | | | |
|---|---|---|---|---|
| Calculated | %: | C,52.91; | H,4.91; | N,6.50. |
| Found | %: | C,52.65; | H,5.04; | N,6.23. |

Specific rotation
$[\alpha]_D^{24}$ -2.3° (c = 1,$H_2O$)

Example 16

(R)-8-Chloro-1-cyclopropyl-6-fluoro-1,4-dihydro-7-[(1-methyl-2-pyrrolidinyl)methoxy]-4-oxoquinoline-3-carboxylic acid

The compound of Example 16 was prepared using (R)-1-methyl-2-pyrrolidinemethanol [$[\alpha]_D^{24}$ +35.7° (c = 1, EtOH)]
Pale yellow crystals, m.p.146-148° C ($CH_3CN$).

Analysis for $C_{19}H_{20}ClFN_2O_4$:

| | | | | |
|---|---|---|---|---|
| Calculated | %: | C,57.80; | H,5.11; | N,7.10. |
| Found | %: | C,57.42; | H,5.19; | N,7.01. |

Specific rotation
$[\alpha]_D^{24}$ +21.1° (c = 1,$CHCl_3$)

Example 17

7-[(3-Azetidinyl)oxy]-8-chloro-1-cyclopropyl-6-fluoro-1,4-dihydro-4-oxoquinoline-3-carboxylic acid hydrochloride

Colorless needles, m.p.198-203° C (MeOH-$Et_2O$).

Analysis for $C_{16}H_{14}ClFN_2O_4 \cdot HCl \cdot 1/2H_2O$:

| | | | | |
|---|---|---|---|---|
| Calculated | %: | C,48.26; | H,4.05; | N, 7.03. |
| Found | %: | C,48.60; | H,4.24; | N, 7.07. |

Example 18

trans-7-[(2-Amino-1-cyclopentyl)oxy]-8-chloro-1-cyclopropyl-6-fluoro-1,4-dihydro-4-oxoquinoline-3-carboxylic acid hydrochloride

Pale brown crystals, m.p.210-215°C (decomp.)(EtOH).

Analysis for $C_{18}H_{18}ClFN_2O_4 \cdot HCl \cdot 3/2H_2O$:

Calculated %: C,48.66; H,4.99; N, 6.31.

Found %: C,48.92; H,4.64; N, 6.34.

Example 19

7-[(2-Aminoethyl)thio]-8-chloro-1-cyclopropyl-6-fluoro-1,4-dihydro-4-oxoquinoline-3-carboxylic acid hydrochloride

A suspension of 1.50g of 8-chloro-1-cyclopropyl-6,7-difluoro-1, 4-dihydro-4-oxoquinoline-3-carboxylic acid in 20ml of N,N-dimethylformamide, 3.11g of potassium carbonate and 1.71g of 2-aminoethanethiol hydrochloride was stirred at room temperature. After stirring for 1 hour, the reaction mixture was adjusted with 10%-hydrochloric acid to pH3, and evaporated. Water was added to the residue, and the solution was adjusted with 10% aqueous sodium hydroxide solution to pH8. The precipitate was collected by filtration, and washed with water and ethanol. The obtained crystals were converted into the hydrochloride in a usual manner, and the resulting salts were recrystallized from methanol to give 1.15g of the desired compound as colorless needles, m.p.222-226°C (decomp.).

Analysis for $C_{15}H_{14}ClFN_2O_3S \cdot HCl \cdot 1/2H_2O$:

Calculated %: C,44.79; H,4.01; N, 6.96.

Found %: C,44.94; H,4.06; N, 6.91.

In the same manner as described in Example 19, the compounds of Examples 20 to 27 were prepared.

Example 20

7-[(3-Aminopropyl)thio]-8-chloro-1-cyclopropyl-6-fluoro-1,4-dihydro-4-oxoquinoline-3-carboxylic acid hydrochloride

Pale yellow needles, m.p.209-214°C (MeOH-$Et_2O$).

Analysis for $C_{16}H_{16}ClFN_2O_3S \cdot HCl \cdot H_2O$:

Calculated %: C,45.19; H,4.50; N, 6.59.

Found %: C,45.12; H,4.58; N, 6.46.

Example 21

8-Chloro-1-cyclopropyl-6-fluoro-1,4-dihydro-7-[[2-(methylamino)ethyl]thio]-4-oxoquinoline-3-carboxylic acid hydrochloride

Pale yellow crystals, m.p.191-196° C (EtOH).

Analysis for $C_{16}H_{16}ClFN_2O_3S \cdot HCl \cdot H_2O$:

Calculated %: C,45.19; H,4.50; N, 6.59.

Found %: C,45.00; H,4.33; N, 6.47.

Example 22

8-Chloro-1-cyclopropyl-7-[[2-(dimethylamino)ethyl]thio]-6-fluoro-1,4-dihydro-4-oxoquinoline-3-carboxylic acid hydrochloride

Pale yellow prisms, m.p.195-199° C (EtOH).

Analysis for $C_{17}H_{18}ClFN_2O_3S \cdot HCl \cdot H_2O$:

Calculated %: C,46.47; H,4.82; N, 6.38.

Found %: C,46.67; H,4.68; N, 6.26.

Example 23

7-[(2-Aminobutyl)thio]-8-chloro-1-cyclopropyl-6-fluoro-1,4-dihydro-4-oxoquinoline-3-carboxylic acid hydrochloride

Colorless prisms, m.p.225-230° C (decomp.)(MeOH).

Analysis for $C_{17}H_{18}ClFN_2O_3S \cdot HCl$:

Calculated %: C,48.46; H,4.55; N, 6.65.

Found %: C,48.12; H,4.86; N, 6.46.

Example 24

7-[(2-Amino-2-methylpropyl)thio]-8-chloro-1-cyclopropyl-6-fluoro-1,4-dihydro-4-oxoquinoline-3-carboxylic acid hydrochloride

Pale yellow crystals, m.p.151-155° C (EtOH-$Et_2O$).

Analysis for $C_{17}H_{18}ClFN_2O_3S \cdot HCl \cdot H_2O$:

Calculated %: C,46.48; H,4.82; N, 6.38.

Found %: C,46.44; H,4.98; N, 6.38.

Example 25

8-Chloro-1-cyclopropyl-6-fluoro-1,4-dihydro-4-oxo-7-[[2-(1-pyrrolidinyl)ethyl]thio]quinoline-3-carboxylic acid hydrochloride

Pale yellow needles, m.p.196-199.5° C (EtOH).

Analysis for $C_{19}H_{20}ClFN_2O_3S \cdot HCl \cdot 2H_2O$:

Calculated %: C,47.21; H,5.21; N, 5.80.

Found %: C,47.49; H,5.02; N, 5.77.

Example 26

8-Chloro-1-cyclopropyl-6-fluoro-1,4-dihydro-7-[(1-methyl-4-piperidinyl)thio]-4-oxoquinoline-3-carboxylic acid

Pale yellow prisms, m.p.209-213° C (MeOH).

Analysis for $C_{19}H_{20}ClFN_2O_3S$:

Calculated %: C,55.54; H,4.91; N, 6.82.

Found %: C,55.48; H,4.91; N, 6.77.

Example 27

trans-7-[(2-Aminocyclopentyl)thio]-8-chloro-1-cyclopropyl-6-fluoro-1,4-dihydro-4-oxoquinoline-3-carboxylic acid hydrochloride

Pale yellow crystals, m.p.185-187° C (decomp.)(MeOH-$Et_2O$).

Analysis for $C_{18}H_{18}ClFN_2O_3S \cdot HCl \cdot 1/4H_2O$:

Calculated %: C,49.38; H,4.49; N, 6.40.

Found %: C,49.18; H,4.59; N, 6.04.

Example 28

8-Chloro-1-cyclopropyl-7-[2-(dimethylamino)ethoxy]-6-fluoro-1,4-dihydro-4-oxoquinoline-3-carboxylic acid hydrochloride

To a solution of 0.50g of 1-cyclopropyl-7-[2-(dimethylamino)-ethoxy]-6-fluoro-1,4-dihydro-4-oxoquinoline-3-carboxylic acid in 2 ml of chlorosulfonic acid was added a trace of iodine, and the mixture was saturated with dry chlorine gas under ice cooling. After stirring for 1.5 hours, the reaction mixture was poured into ice-water, and adjusted with 10% aqueous sodium hydroxide solution to pH8. The whole was extracted with chloroform. The extract was dried and evaporated. Isopropylether was added to the residue, and the precipitate was collected by filtration. The obtained crystals were converted into the hydrochloride in a usual manner, and the resulting salt was recrystallized from methanol to give 0.20g of desired compound as pale yellow needles, m.p.199-202°C.

Analysis for $C_{17}H_{18}ClFN_2O_4 \cdot HCl$:

Calculated %: C,50.39; H,4.73; N, 6.91.

Found %: C,50.40; H,4.72; N, 6.85.

In the same manner as described in Example 28, the compounds of Examples 29 to 41 were prepared.

Example 29

7-(2-Aminopropoxy)-8-chloro-1-cyclopropyl-6-fluoro-1,4-dihydro-4-oxoquinoline-3-carboxylic acid hydrochloride

Colorless crystals, m.p.218-222°C (MeOH-$Et_2O$).

Analysis for $C_{16}H_{16}ClFN_2O_4 \cdot HCl$:

Calculated %: C,49.12; H,4.38; N, 7.16.

Found %: C,49.04; H,4.18; N, 7.15.

Example 30

7-(2-Aminobutoxy)-8-chloro-1-cyclopropyl-6-fluoro-1,4-dihydro-4-oxoquinoline-3-carboxylic acid hydrochloride

Gray crystals, m.p.210-212°C (EtOH-$Et_2O$).

Analysis for $C_{17}H_{18}ClFN_2O_4 \cdot HCl$:

Calculated %: C,50.39; H,4.73; N, 6.91.

Found %: C,50.17; H,4.92; N, 6.73.

Example 31

8-Chloro-1-cyclopropyl-7-[2-(dimethylamino)propoxy]-6-fluoro-1,4-dihydro-4-oxoquinoline-3-carboxylic acid

hydrochloride

Pale yellow crystals, m.p.179-182° C (decomp.)(EtOH-$Et_2O$).
IR spectrum $\nu$ (KBr) $cm^{-1}$: 1730 (COOH), 1608 (C=O)
NMR spectrum $\delta$ (DMSO-$d_6$) ppm: 1.03-1.38 (4H,m), 1.51 (3H,d,J=7Hz), 2.84 (6H,s), 3.61-4.02 (1H,m), 4.24-4.55 (1H,m), 4.63 (2H,d,J=5Hz), 8.11 (1H,d,J=11Hz), 8.84 (1H,s).

Example 32

7-(2-Amino-2-methylpropoxy)-8-chloro-1-cyclopropyl-6-fluoro-1,4-dihydro-4-oxoquinoline-3-carboxylic acid sulfate

Colorless crystals, m.p.212-214° C (EtOH-$Et_2O$).

Analysis for $C_{17}H_{18}ClFN_2O_4 \cdot H_2SO_4 \cdot H_2O$:

Calculated %: C,42.11; H,4.57; N, 5.78.

Found %: C,41.93; H,4.43; N, 5.78.

Example 33

7-(2-Amino-1-methylethoxy)-8-chloro-1-cyclopropyl-6-fluoro-1,4-dihydro-4-oxoquinoline-3-carboxylic acid hydrochloride

Pale yellow crystals, m.p.229-233° C (decomp.)(MeOH).

Analysis for $C_{16}H_{16}ClFN_2O_4 \cdot HCl$:

Calculated %: C,49.12; H,4.38; N, 7.16.

Found %: C,49.10; H,4.61; N, 7.09.

Example 34

(R)-7-(2-Aminopropoxy)-8-chloro-1-cyclopropyl-6-fluoro-1,4-dihydro-4-oxoquinoline-3-carboxylic acid hydrochloride

The compound of Example 34 was prepared using (R)-7-(2-aminopropoxy)-1-cyclopropyl-6-fluoro-1,4-dihydro-4-oxoquinoline-3-carboxylic acid hydrochloride

[$[\alpha]_D^{22.3}$ -7.4° (c=1,$H_2O$)].
Pale brown crystals, m.p.228-233° C (decomp.)(MeOH-$Et_2O$).
IR spectrum $\nu$ (KBr) $cm^{-1}$: 1728 (COOH), 1608 (C=O)
NMR spectrum $\delta$ (DMSO-$d_6$) ppm: 0.96-1.50 (4H,m), 1.41 (3H,d,J=7Hz), 3.52-3.84 (1H,m), 4.26-4.62 (1H,m), 4.39 (2H,dd,J=5.5,0.5Hz), 8.13 (1H,d,J=11Hz), 8.84 (1H,s) Specific rotation

$[\alpha]_D^{22.3}$ -7.5° (c=1, $H_2O$)

Example 35

(S)-7-(2-Aminopropoxy)-8-chloro-1-cyclopropyl-6-fluoro-1,4-dihydro-4-oxoquinoline-3-carboxylic acid hydrochloride

The compound of Example 35 was prepared using (S)-7-(2-aminopropoxy)-1-cyclopropyl-6-fluoro-1,4-dihydro-4-oxoquinoline-3-carboxylic acid hydrochloride

$[[\alpha]_D^{22.3}$ $+7.8^\circ$ $(c=1, H_2O)]$.
Pale yellow crystals, m.p.223-228 °C (decomp.)($MeOH-Et_2O$).
IR spectrum ν (KBr) $cm^{-1}$: 1732 (COOH), 1616 (C=O)
NMR spectrum δ ($DMSO-d_6$) ppm: 0.96-1.50 (4H,m), 1.40 (3H,d,J=7Hz), 3.50-3.90 (1H,m), 4.20-4.60 (1H,m), 4.37 (2H,dd,J=5.5,0.5Hz), 8.13 (1H,d,J=11Hz), 8.84 (1H,s)
Specific rotation

$[\alpha]_D^{23.8}$ $+3.1^\circ$ (c=1,MeOH)

Example 36

8-Chloro-1-cyclopropyl-6-fluoro-1,4-dihydro-4-oxo-7-[2-(1-pyrrolidinyl)ethoxy]quinoline-3-carboxylic acid

Pale yellow needles, m.p.136-137 °C (AcOEt).

Analysis for $C_{19}H_{20}ClFN_2O_4$:
Calculated %: C,57.80; H,5.11; N, 7.10.
Found %: C,57.80; H,5.12; N, 7.22.

Example 37

(S)-8-Chloro-1-cyclopropyl-6-fluoro-1,4-dihydro-4-oxo-7-[(2-pyrrolidinyl)methoxy]quinoline-3-carboxylic acid hydrochloride

The compound of Example 37 was prepared using (S)-1-cyclopropyl-6-fluoro-1,4-dihydro-4-oxo-7-[(2-pyrrolidinyl)methoxy]quinoline-3-carboxylic acid
$[[\alpha]_D^{23}$ $-2.8^\circ$ (c=1, 0.1N-HCl)]
Pale brown crystals, m.p.202-205 °C ($MeOH-Et_2O$).

Analysis for $C_{18}H_{18}ClFN_2O_4 \cdot HCl \cdot H_2O$:
Calculated %: C,49.67; H,4.86; N, 6.44.
Found %: C,49.82; H,4.79; N, 6.40.

Specific rotation
$[\alpha]_D^{24}$ $+16.9^\circ$ $(c=1, H_2O)$

Example 38

8-Chloro-1-cyclopropyl-6-fluoro-1,4-dihydro-4-oxo-7-[(3-pyrrolidinyl)oxy]quinoline-3-carboxylic acid hydrochloride

Pale brown crystals, m.p.253.5-254.5° C (decomp.)($H_2O$-EtOH).

Analysis for $C_{17}H_{16}ClFN_2O_4 \cdot HCl$:

Calculated %: C,50.64; H,4.25; N, 6.95.

Found %: C,50.45; H,4.41; N, 6.74.

Example 39

8-Chloro-1-cyclopropyl-6-fluoro-1,4-dihydro-7-[(1-methyl-3-pyrrolidinyl)oxy]-4-oxoquinoline-3-carboxylic acid hydrochloride

Pale yellow crystals, m.p.193-196° C (EtOH).

Analysis for $C_{18}H_{18}ClFN_2O_4 \cdot HCl \cdot H_2O$:

Calculated %: C,49.67; H,4.86; N, 6.44.

Found %: C,49.63; H,4.81; N, 6.36.

Example 40

8-Chloro-1-cyclopropyl-6-fluoro-1,4-dihydro-7-[(1-methyl-4-piperidinyl)oxy]-4-oxoquinoline-3-carboxylic acid

Pale brown prisms, m.p.185-189° C (EtOH).

Analysis for $C_{19}H_{20}ClFN_2O_4$:

Calculated %: C,57.80; H,5.11; N, 7.10.

Found %: C,57.64; H,5.28; N, 7.02.

Example 41

8-Chloro-1-cyclopropyl-6-fluoro-1,4-dihydro-4-oxo-7-[(3-piperidinyl)oxy]quinoline-3-carboxylic acid

Pale brown crystals, m.p.179-181° C (decomp.)($CHCl_3$-MeOH).

Analysis for $C_{18}H_{18}ClFN_2O_4 \cdot 2H_2O$:

Calculated %: C,51.87; H,5.32; N, 6.72.

Found %: C,51.88; H,5.18; N, 6.73.

Example 42

8-Chloro-1-cyclopropyl-7-[2-(dimethylamino)-2-methylpropoxy]-6-fluoro-1,4-dihydro-4-oxoquinoline-3-carboxylic acid hydrochloride

A suspension of 0.35g of 7-(2-amino-2-methylpropoxy)-8-chloro-1-cyclopropyl-6-fluoro-1,4-dihydro-4-oxoquinoline-3-carboxylic acid hydrochloride, 1.62ml of 37%-formalin and 1.63ml of formic acid was stirred for 8 hours at 90°C. The reaction mixture was evaporated. Water was added to the residue, and the solution was adjusted with 10% aqueous sodium hydroxide solution to pH8. The whole was extracted with chloroform. The extract was dried and evaporated. The residue was converted into the hydrochloride in a usual manner, and the resulting salt was recrystallized from a mixture of ethanol and ether to give 0.16g of the desired compound as pale yellow crystals, m.p.174-179°C(decomp.).
IR spectrum $\nu$ (KBr) $cm^{-1}$: 1730 (COOH), 1620 (C=O)
NMR spectrum $\delta$ ($DMSO-d_6$) ppm: 0.84-1.42 (4H,m), 1.53 (6H,s), 2.84(6H,s), 4.20-4.60 (3H,m), 8.14 (1H,d,J=10.5Hz), 8.84 (1H,s)

Example 43

| Tablet formulation | (mg) |
|---|---|
| Compound of Example 13 | 100 |
| Lactose | q.s. |
| Starch | 20 |
| Magnesium Stearate | 1 |
| Hydroxypropylmethylcellulose | 3.4 |
| Polyethyleneglycol 6000 | 0.2 |
| Titanium oxide | 0.4 |
| | 170 mg |

Example 44

| Capsule formulation | (mg) |
|---|---|
| Compound of Example 29 | 100 |
| Lactose | q.s. |
| Carboxymethylcellulose Calcium | 15 |
| Hydroxypropylcellulose | 2 |
| Magnesium Stearate | 1 |
| | 160 mg |

Example 45

| Granule formulation | (mg) |
|---|---|
| Compound of Example 32 | 100 |
| Lactose | q.s. |
| D-Mannitol | 400 |
| Hydroxypropylcellulose | 5 |
| Talc | 2 |
| | 1000 mg |

Example 46

| Injection formulation | (mg) |
|---|---|
| Compound of Example 35 | 50 |
| Glucose | 1000 |
| Hydrochloric acid | q.s. |
| Water for injection | q.s. |
| | 20 ml |

Example 47

| Suppository formulation | (mg) |
|---|---|
| Compound of Example 39 | 50 |
| Hard fat | 1350 |
| | 1400 mg |

It is to be understood that the invention is not to be limited to the exact details of operation, or to the exact compositions, methods, procedures, or embodiments shown and described, as modifications and equivalents will be apparent to one skilled in the art, and the invention is therefore to be limited only by the full scope of the appended claims.

## Claims

(1) A quinoline-3-carboxylic acid compound represented by the formula (I);

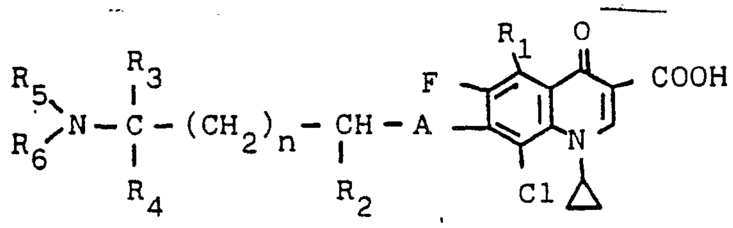

(I)

wherein $R_1$ is a hydrogen atom or an amino group; $R_2$ is a hydrogen atom or a lower-alkyl group, or $R_2$ combined with $R_4$ is an alkylene group having 1 through 4 methylenes; $R_3$ and $R_4$ are the same or different and each is a hydrogen atom or a lower-alkyl group, or $R_3$ combined with $R_4$ is an alkylene group having 2 through 6 methylenes; $R_5$ and $R_6$ are the same or different and each is a hydrogen atom or a lower-alkyl group, or $R_5$ combined with $R_2$ is an alkylene group having 1 through 4 methylenes, or $R_5$ combined with $R_3$, together with the neighboring nitrogen atom, is a 5- through 7- membered ring system which may be substituted, or $R_5$ and $R_6$, together with the neighboring nitrogen atom, is a 5-through 7- membered ring system which may be substituted; A is an oxygen atom or a sulfur atom; n is selected from 0 through 3, or a pharmacologically-acceptable salt thereof.

(2) A compound of claim 1 which is 7-(2-aminoethoxy)-8-chloro-1-cyclopropyl-6-fluoro-1,4-dihydro-4-oxoquinoline-3-carboxylic acid or a pharmacologically-acceptable salt thereof.

(3) A compound of claim 1 which is 7-(2-aminopropoxy)-8-chloro-1-cyclopropyl-6-fluoro-1,4-dihydro-4-oxoquinoline-3-carboxylic acid or a pharmacologically-acceptable salt thereof.

(4) A compound of claim 1 which is (R)-7-(2-aminopropoxy)-8-chloro-1- cyclopropyl-6-fluoro-1,4-dihydro-4- oxoquinoline-3-carboxylic acid or a pharmacologically-acceptable salt thereof.

(5) A compound of claim 1 which is (S)-7-(2-aminopropoxy)-8-chloro-1- cyclopropyl-6-fluoro-1, 4-dihydro-4-oxoquinoline-3-carboxylic acid or a pharmacologically-acceptable salt thereof.

(6) A compound of claim 1 which is 7-(2-amino-2-methylpropoxy)-8-chloro-1-cyclopropyl-6-fluoro-1,4-dihydro-4-oxoquinoline-3-carboxylic acid or a pharmacologically-acceptabe salt thereof.

(7) A compound of claim 1 which is 7-[(1-aminocyclopropyl)-methoxy]-8- chloro-1-cyclopropyl-6-fluoro-1,4-dihydro-4-oxoquinoline-3-carboxylic acid or a pharmacologically-acceptable salt thereof.

(8) A compound of claim 1 which is 7-(2-amino-1-methylethoxy)-8-chloro-1-cyclopropyl-6-fluoro-1,4-dihydro-4-oxoquinoline-3-carboxylic acid or a pharmacologically-acceptable salt thereof.

(9) A compound of claim 1 which is trans-7-[(2-amino-1-cyclopentyl)oxy]-8-chloro-1-cyclopropyl-6-fluoro-1, 4-dihydro-4-oxoquinoline-3-carboxylic acid or a pharmacologically-acceptable salt thereof.

(10) A compound of claim 1 which is 8-chloro-1-cyclopropyl-6-fluoro- 1,4-dihydro-7-[(1-methyl-3-pyrrolidinyl)oxy]-4-oxoquinoline-3-carboxylic acid or a pharmacologically-aceptable salt thereof.

(11) A compound of claim 1 which is 8-chloro-1-cyclopropyl-6-fluoro- 1,4-dihydro-7-[(1-methyl-4-piperidinyl)oxy]-4-oxoquinoline-3-carboxylic acid or a pharmacologically-acceptable salt thereof.

(12) A compound of claim 1 which is 7-[(2-aminoethyl)thio]-8-chloro- 1-cyclopropyl-6-fluoro-1,4-dihydro-4-oxoquinoline-3-carboxylic acid or a pharmacologically-acceptable salt thereof.

(13) A compound of claim 1 which is 5-amino-7-(2-aminopropoxy)-8-chloro-1-cyclopropyl-6-fluoro-1,4-dihydro-4-oxoquinoline-3-carboxylic acid or a pharmacologically-acceptable salt thereof.

(14) An antibacterial agent useful for the treatment of bacterial infection, comprising an effective amount of a quinoline-3-carboxylic acid compound of claim 1.

(15) A pharmaceutical composition useful for the treatment of bacterial infection, comprising an effective amount of a quinoline-3-carboxylic acid compound of claim 1 and a pharmaceutically-acceptable carrier or diluent.

(16) A method for the treatment of a bacterial infection, comprising the step of administering an effective amount of a quinoline-3-carboxylic acid compound of claim 1, or a pharmaceutical composition comprising the same, to a subject in need thereof.

(17) A process for preparing a quinoline-3-carboxylic acid compound represented by the formula (I);

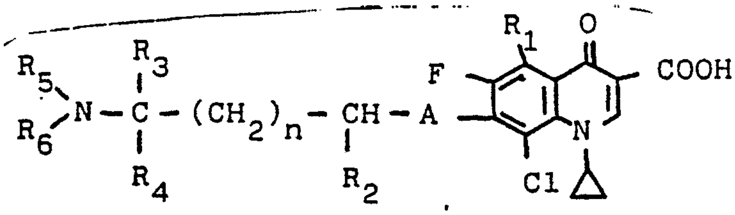

(I)

wherein $R_1$ is a hydrogen atom or an amino group; $R_2$ is a hydrogen atom or a lower-alkyl group, or $R_2$ combined with $R_4$ is an alkylene group having 1 through 4 methylenes; $R_3$ and $R_4$ are the same or different and each is a hydrogen atom or a lower-alkyl group, or $R_3$ combined with $R_4$ is an alkylene group having 2 through 6 methylenes; $R_5$ and $R_6$ are the same or different and each is a hydrogen atom or a lower-alkyl group, or $R_5$ combined with $R_2$ is an alkylene group having 1 through 4 methylenes, or $R_5$ combined with $R_3$, together with the neighboring nitrogen atom, is a 5-through 7-membered ring system which may be substituted, or $R_5$ and $R_6$, together with the neighboring nitrogen atom, is a 5-through 7- membered ring system which may be substituted; A is an oxygen atom or a sulfur atom; n is selected from 0 through 3, or a pharmacologically-acceptable salt thereof, which comprises reacting a 7-halogenoquinoline-3-carboxylic acid represented by the formula (II);

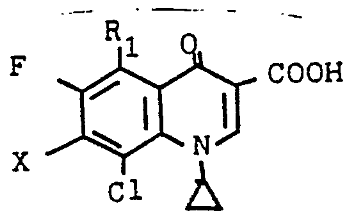

(II)

wherein $R_1$ has the same meaning as that described above, while X is a halogen atom, with an alcohol or thioalcohol compound represented by the formula (III);

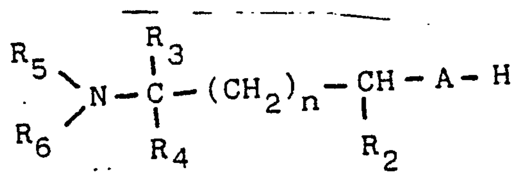

(III)

wherein $R_2$, $R_3$, $R_4$, $R_5$, $R_6$, A and n each has the same meaning as described above.

(18) A process for preparing a quinoline-3-carboxylic acid compound represented by the formula (I);

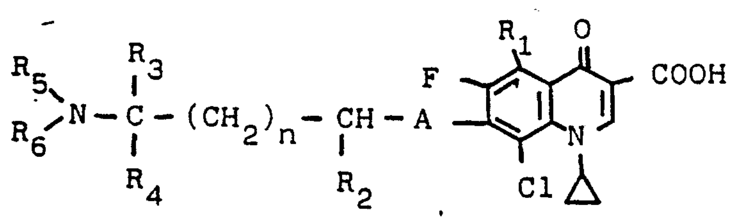

(I)

wherein $R_1$ is a hydrogen atom or an amino group; $R_2$ is a hydrogen atom or a lower-alkyl group, or $R_2$ combined with $R_4$ is an alkylene group having 1 through 4 methylenes; $R_3$ and $R_4$ are the same or different and each is a hydrogen atom or a lower-alkyl group, or $R_3$ combined with $R_4$ is an alkylene group having 2 through 6 methylenes; $R_5$ and $R_6$ are the same or different and each is a hydrogen atom or a lower-alkyl group, or $R_5$ combined with $R_2$ is an alkylene group having 1 through 4 methylenes, or $R_5$ combined with $R_3$, together with the neighboring nitrogen atom, is a 5- through 7- membered ring system which may be substituted, or $R_5$ and $R_6$, together with the neighboring nitrogen atom, is a 5-through 7- membered ring system which may be substituted; A is an oxygen atom or a sulfur atom; n is selected from 0 through 3, or a pharmacologically-acceptable salt thereof, which comprises reacting a compound represented by the

formula (IV);

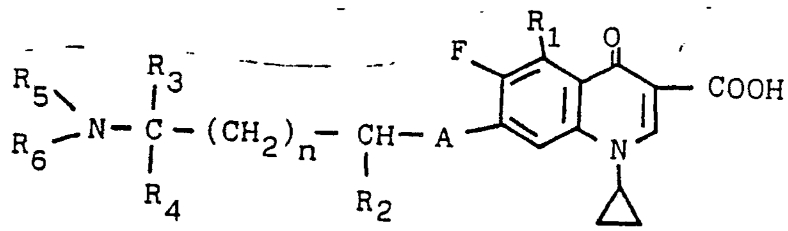

(IV)

wherein $R_1$, $R_2$, $R_3$, $R_4$, $R_5$, $R_6$, A and n each has the same meaning as described above, with a chlorinating agent to introduce a chlorine atom into the 8 position thereof.

European Patent Office

# PARTIAL EUROPEAN SEARCH REPORT

which under Rule 45 of the European Patent Convention shall be considered, for the purposes of subsequent proceedings, as the European search report

Application number
EP 89106778.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A - 0226961 (WARNER-LAMBERT CO.) * claims 1,4 *; & JP - A - 62 187 459 (Cat. D) --- | 1,14, 15 | C07D215/56<br>A61K31/435 |
| A | EP - A - 0167763 (BAYER AG) * claims 1,12; abstract *; & JP - A - 61 1667 (Cat. D) --- | 1,14, 15 | |
| A | EP - A - 0119779 (RIKER LABORATORIES INC.) * claims 1,10; abstract * --- | 1,14, 15 | |
| A | PATENT ABSTRACTS OF JAPAN, vol. 9, no. 228 (C-303)(1951), 13th September 1985; & JP - A - 60 89480 (DAINIPPON SEIYAKU, K.K.) 20.05.85 --- | 1,14, 15 | |
| A | EP - A - 0132845 (DAINIPPON PHARMACEUTICAL CO., LTD.) * abstract * --- | 1,14, 15 | |
| A | GB - A - 1122323 (IMPERIAL CHEMICAL INDUSTRIES, LTD.)* page 1, lines 1-23; page 2, lines 67-82 * --- | 1,14, 15 | TECHNICAL FIELDS SEARCHED (Int. Cl.4)<br>C07D215/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-15,17,18

Claims searched incompletely: -

Claims not searched: 16

Reason for the limitation of the search: Method for treatment of the human or animal body by surgery or therapv (Art. 52(4) EPC)

| Place of search | Date of completion of the search | Examiner |
|---|---|---|
| Berlin | 10.07.1989 | C.V.F. HASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document